# EUROPEAN PATENT APPLICATION

(11) **EP 4 406 472 A1**
(43) Date of publication of application: **31.07.2024**
(21) Application number: 24154250.5
(22) Date of filing: 26.01.2024
(51) Int. Cl.: A61B 3/00, A61B 3/113, A61B 5/16

(54) **EYE-TRACKING DEVICE AND METHOD FOR TESTING VISUAL PROCESSING CAPABILITIES**

(30) Priority: 27.01.2023 US 202318102263
(71) Applicant: King Faisal Specialist Hospital & Research Centre, Riyadh, 11211 (SA)
(72) Inventor: ALDHALAAN, Hesham, 11211 Riyadh (SA); ALARIFI, Hana, 11211 Riyadh (SA); ALARIFI, Jhan, 11211 Riyadh (SA); ALABDULAZIZ, Reem, 11211 Riyadh (SA)
(74) Representative: Hartig, Michael

(57) **Abstract**

A device and method for testing visual processing capabilities of a subject is provided. The device comprises a display unit configured to display a visual stimulus picture to the subject and a tracking unit configured to perform eye tracking to record gaze data of the subject during display of the visual stimulus picture. The gaze data includes one or more time parameters associated with the gaze of the subject to an area in the visual stimulus picture. The device further comprises a processing unit configured to evaluate the one or more time parameters in the recorded gaze data, the one or more time parameters capable of indicating an autism status of the subject.

## Description

### TECHNICAL FIELD

The present invention relates to a device and method for testing visual processing capabilities of a subject. In particular, the present invention relates to a device and method for testing visual processing capabilities of the subject to indicate an autism status of the subject.

### BACKGROUND

Autism is a neurodevelopmental disorder characterized by differences in social and communicative development, sensory alterations, and restrictions in behavior and interest. Currently, an autism status of a person is diagnosed by a clinician based on symptoms and a developmental history of each individual subject. However, no objective tests are available to support the medically trained personnel in diagnostic decision making. Hence, certain symptoms indicating an autism status of the subject, such as alterations in the visual processing capabilities of the subject, may be overlooked or misinterpreted by the clinician, or may be recognizable by the clinician only after a certain age of the subject. As consequence, therapeutic measures and measures promoting the subject's social and communicative development may be belated.

Other techniques of the state-of-the-art may employ testing the subject's blood for biomarkers to predict an autism status of the subject. However, such techniques are invasive, may lack accuracy, may not be readily available, and no validated biomarker is currently available.

In view of this, a problem may be providing a device and method that enable an objective, accurate, non-invasive, readily available, and user-friendly test to support decision-making in diagnosing an autism status of a subject. A further problem may be predicting an autism status of the subject. A further problem may be providing a device and method for testing visual processing capabilities of the subject to provide an indication for an autism status of the subject. A further problem may be increasing the accuracy of a device and method for testing the visual processing capabilities of the subject, thus increasing the accuracy for providing an indication for an autism status of the subject.

### SUMMARY OF INVENTION

At least one of the above problems is solved by the subject matter of the independent claims. Embodiments are given by the features of the dependent claims.

A device for testing visual processing capabilities of a subject is provided. The device may comprise a display unit configured to display a visual stimulus picture to the subject and a tracking unit configured to perform eye tracking to record gaze data of the subject during display of the visual stimulus picture. The gaze data may include one or more time parameters associated with the gaze of the subject to an area in the visual stimulus picture. The device may further comprise a processing unit configured to evaluate the one or more time parameters in the recorded gaze data, wherein the one or more time parameters may be capable of indicating an autism status of the subject.

Correspondingly, a method for testing visual processing capabilities of a subject is provided. The method may comprise displaying a visual stimulus picture to the subject and performing eye tracking to record gaze data of the subject during display of the visual stimulus picture. The gaze data may include one or more time parameters associated with the gaze of the subject to an area in the visual stimulus picture. The method may further comprise evaluating the one or more time parameters in the recorded gaze data, wherein the one or more time parameters may be capable of indicating an autism status of the subject.

An autism status of the subject may be associated with an atypical gaze behavior when the subject views a visual stimulus picture. The gaze behavior of the subject may be reflected in or captured by one or more time parameters associated with the gaze of the subject to a specific area in the visual stimulus picture. Hence, the one or more time parameters may be capable of indicating an autism status of the subject. When eye tracking is performed to record gaze data of the subject during display of the visual stimulus picture, the gaze data may include the one or more time parameters. Thereafter, the one or more time parameters may be evaluated and may serve as indication for an autism status of the subject. For example, a significant deviation of the evaluated one or more time parameters from predetermined standard values may reflect an atypical gaze behavior associated with a potential autism status of the subject.

Performing eye tracking to record gaze data of the subject during display of the visual stimulus picture and evaluating the one or more time parameters included in the recorded gaze data may advantageously provide a non-invasive, user-friendly, and accurate testing technique to support decision-making in diagnosing an autism status of the subject.

The subject may be an infant, child, or adult, e. g. with an age in the range of several months to several years. The device and method for testing visual capabilities of the subject may enable an indication or prediction for an autism status of the subject already from an early age of the subject.

Preferably at least two time parameters may be evaluated. Thereby, the testing accuracy (sensitivity and specificity) may be improved.

The display unit may comprise a screen to display the visual stimulus picture to the subject. The tracking unit may comprise an optical system, such as one or more cameras, to capture the gaze of the subject during display of the visual stimulus picture to the subject, i.e., recording gaze data of the subject.

The processing unit and the tracking unit may be communicatively connected. More specifically, the tracking unit may be configured to record the gaze data and transmit the gaze data to the processing unit. The processing unit may be configured to receive the recorded gaze data from the tracking unit for further processing. For example, the processing unit and the tracking unit may each comprise a bus for cable-based or wireless communication. The processing unit and the tracking unit may use any transfer protocol to transfer the gaze data, such as the Hypertext Transfer Protocol.

Evaluating the one or more time parameters in the recorded gaze data may refer to processing or analyzing the recorded gaze data in order to provide a value for each of the one or more time parameters. More specifically, the gaze data may be provided as raw data comprising a time series of gaze coordinates and corresponding time instances, and the area in the visual stimulus picture may be represented by a plurality of (pixel) coordinates. Evaluating a time parameter of the one or more time parameters may then mean that the gaze coordinates are compared to the coordinates of the area in the visual stimulus picture. When a gaze coordinate of the time series matches or is close to a coordinate of the area in the visual stimulus picture, the corresponding time instance of the gaze coordinate may be used for calculating the value of the time parameter. For example, time instances may be counted during which a gaze coordinate of the time series matches or is close to a coordinate of the area in the visual stimulus picture. The number of counted time instance may then be multiplied with a time interval between the time instances to provide the value for the time parameter, i.e., an overall or summed dwell time of the gaze of the subject to the area in the visual stimulus picture. Alternatively, the time instances may be gathered to sets of time instances during which the gaze of the subject continuously stays within the area in the visual stimulus picture. A value for the time parameter may then be provided by calculating an average dwell time of the gaze of the subject to the area in the visual stimulus picture from the sets of time instances. In another example, evaluating a time parameter of the one or more time parameters may refer to searching the time series from the start of the display of the visual stimulus picture to the subject for the time instance when the corresponding gaze coordinate first matches or is close to a coordinate of the area in the visual stimulus picture. This time instance may then provide a value for the time parameter, i.e., a time between the display of the visual stimulus picture to the subject and a fixation of the gaze of the subject to the area in the visual stimulus picture. Evaluating the one or more time parameters may also refer to calculating an average value for each of the one or more time parameters from different time series of gaze coordinates and time instances recorded during the display of different visual stimulus pictures. Further, evaluating the one or more time parameters may also refer to calculating an average value for each of the one or more time parameters from recorded gaze data of different subjects. This may also enable determining standard values for the one or more time parameters, e.g. from subjects pre-known or post-verified to not having an autism status.

The processing unit may comprise a processor and/or graphical processing unit for evaluating the one or more time parameters included in the gaze data. The processing unit may further comprise memory for storing the recorded gaze data and the evaluated one or more time parameters. The processing unit may further store program code comprising instructions for performing the evaluation. In an example, the processing unit may be a personal computer or a handheld computing device. Additionally, or alternatively, the processing unit may be a server. In an example, the processing unit may be in communication with a further control device available to medically trained personnel, e.g., a clinician, supervising the testing setup. The control device may be configured to provide the clinician with the evaluation of the one or more time parameters, who may then decide to diagnose an autism status of the subject based on the evaluation of the one or more time parameters.

In an embodiment, the one or more time parameters may include a time between the display of the visual stimulus picture to the subject and a fixation of the gaze of the subject to the area in the visual stimulus picture. Additionally, or alternatively, the one or more time parameters may include a dwell time of the gaze of the subject on the area in the visual stimulus picture.

According to an embodiment, the one or more time parameters may be associated with the subject viewing an area in the visual stimulus picture located in the left hemifield of the subject's visual field.

A person, on average, may usually focus first and for a longer duration on an area in the visual stimulus picture that is on the left hemifield of their visual field, i.e., they display so-called "left visual field bias". This left visual field bias may particularly occur during face perception, which may be attributed to the right hemispheric dominance during face processing. For a subject with an autism status, this left visual field bias may be less pronounced or even non-existing. Hence, left visual field bias is a metric that may be particularly suitable for providing an indication for a potential autism status of the subject. In other words, evaluating the one or more time parameters that are associated with the subject viewing an area of the visual stimulus picture located in the left hemifield of the subject's visual field in the recorded gaze data may enable extracting information on left visual field bias from the recorded gaze data and hence, may be particularly suitable for indicating an autism status of the subject. On that basis, the testing accuracy may be increased.

In a further embodiment, the visual stimulus picture may include an image of a face, preferably the face of a human or the face of a cartoon animal. The face may have a gaze direction directly towards the observer, i.e., the subject. The gaze behavior of a subject having an autism status may be altered when the subject views social information such as faces, i.e., a subject having an autism status may show deviations during face perception. Hence, an image of a face depicted in the visual stimulus picture may be particularly suitable for reflecting the atypical gaze behavioral profile and hence, may be particularly suitable for providing an indication for a potential autism status of the subject. Using the face of a cartoon animal may enable eliciting both of similarities and differences compared with the perception of human faces.

In an embodiment, the one or more time parameters may be associated with the subject viewing an area in an eye region of a face depicted in the visual stimulus picture. An average person may tend to focus first and for longer duration on the central area, specifically an area in an eye region, of face depicted the visual stimulus picture. This tendency, however, may be less pronounced or even completely depleted for a subject having an autism status. Hence, evaluating one or more time parameters that are associated with the subject viewing an area in an eye region of a face depicted in the visual stimulus picture, hereinafter also called as the metric "attention to eyes", may be particularly advantageous for providing an indication for a potential autism status of the subject.

In a further embodiment, the one or more time parameters may be associated with the subject viewing an area in a region of an image of one or more objects or events depicted in the visual stimulus picture. In an example, the object may be a toy. An event may be the appearance of a light signal. Additionally, or alternatively, an event may be the appearance or movement of the image of the one or more objects. A face depicted in the visual stimulus picture may have a gaze direction towards one of the one or more objects or events.

So-called "joint attention" is an essential metric for social and language development among children. Joint attention includes the behavioral pattern of reacting to another person's gaze direction by viewing the same objects and events as the other person. In typical infant development, joint attention starts developing during the second half of the first year. Joint attention, however, is impaired in a subject having an autism status. Hence, detecting impairments in joint attention may be particularly suitable for providing an indication for an autism status of the subject at an early stage. In other words, evaluating the one or more time parameters associated with the subject viewing an area in a region of an image of one or more objects or events depicted in the visual stimulus, preferably with a face depicted in the visual stimulus picture having a gaze direction towards one of the one or more objects or events, may be particularly suitable for providing an indication for a potential autism status of the subject. On that basis, testing accuracy may be increased.

In an embodiment, the face depicted in the visual stimulus picture may change its gaze direction from one of the one or more objects or events to another. More specifically, the visual stimulus picture may be an animation of a face that changes its gaze direction from one of the one or more objects or events to another during display of the visual stimulus picture to the subject. This may be particularly advantageous for testing for delays in reflexive joint attention.

The above-described metrics of left visual field bias, attention to eyes, and joint attention may be evaluated individually or jointly for providing an indication for a potential autism status of the subject. Combining the above-described metrics may advantageously further increase the testing accuracy. In other words, evaluating at least two time parameters - each associated with a different metric selected from a group of metrics including left visual field bias, attention to eyes, and joint attention - may further increase the testing accuracy.

In yet another embodiment, the display unit may be configured to consecutively display a plurality of visual stimulus pictures to the subject, wherein each of the plurality of visual stimulus pictures is displayed to the subject for a predetermined duration, e. g. several seconds.

Correspondingly, the method for testing visual capabilities of the subject may further comprise consecutively displaying a plurality of visual stimulus pictures to the subject, wherein each of the plurality of visual stimulus pictures is displayed to the subject for a predetermined duration.

Preferably, each of the plurality of visual stimulus pictures may be displayed to the subject for a duration in the range of 0.5 seconds to 20 seconds, more preferably 1 second to 10 seconds, and more preferably 3 seconds to 5 seconds. The visual stimulus pictures of the plurality of visual stimulus pictures may differ in appearance of depicted faces, type or property of depicted objects or events, or gaze direction of the depicted face. This may allow excluding systematic errors and improving testing accuracy.

In an embodiment, the processing unit may be configured to predict the autism status of the subject. Correspondingly, the method for testing visual capabilities of the subject may further comprise predicting the autism status of the subject.

For example, the processing unit may be configured to predict the autism status of the subject based on comparing the evaluated one or more time parameters in the recorded gaze data to standard values. The processing unit may store the standard values in its memory and may further store program code comprising instructions for performing the comparison. Correspondingly, the method for testing visual capabilities of the subject may further comprise predicting the autism status of the subject based on, preferably automatically, comparing the evaluated one or more time parameters in the recorded gaze data to standard values.

For example, the predetermined standard values may be previously obtained from subjects that were tested for signs of autism and were found to not having an autism status. The evaluated one or more time parameters in the recorded gaze data of the subject may be compared to the standard values. Finding a significant deviation of the evaluated one or more time parameters from the standard values, e.g. the evaluated one or more time parameters being significantly higher or lower than predetermined standard or threshold values, may then serve as basis for the prediction of the autism status of the subject. Alternatively, standard values may be obtained from subjects known to having an autism status. When the evaluated one or more time parameters are close to the standard values, i.e. may be in a specific range including the standard value, the subject may be labeled as likely having an autism status.

This prediction by the processing unit may advantageously support diagnosis of an autism status of the subject. For example, a clinician may be provided with the evaluated one or more time parameters in addition to the prediction from the processing unit, which may support his or hers decision-making in diagnosing an autism status of the subject.

Additionally, or alternatively, the processing unit may be configured to perform a machine learning algorithm to predict the autism status of the subject based on the evaluation of the one or more time parameters in the recorded gaze data. Correspondingly, the method for testing visual capabilities of the subject may further comprise performing a machine learning algorithm to predict the autism status of the subject based on the evaluation of the one or more time parameters in the recorded gaze data. Machine learning (ML) approaches such as the Random Forest Model (RFM), Decision Tree, Artificial Neural Network, and Long Term Short Term Memory (LSTM) may be used to categorise Autism Spectrum Disorder (ASD).

The machine learning algorithm may be trained with gaze data previously recorded from a plurality of subjects, who are subdivided into two groups, i.e., subjects found to having an autism status and subjects found to not having an autism status (i.e., neurotypical). The processing unit may then employ the trained machine learning algorithm to classify the tested subject as subject with or without an autism status. This machine learning classification may serve as prediction for a potential autism status of the subject. The prediction result may be provided to the clinician in the form of a classifier reflecting a likelihood of the subject having (or not having) an autism status. This may provide an objective measure for supporting decision-making in diagnosing an autism status of the subject. In an example, the subject may be diagnosed with an autism status when the classifier exceeds a certain probability threshold.

In an example, the machine learning algorithm is a Random Forest model based on individual variable features including the one more time parameters. The above-described metrics of left visual field bias, attention to eyes, and joint attention may be used jointly in the machine learning classification. Thereby, the accuracy for predicting an autism status may be increased.

### BRIEF DESCRIPTION OF THE DRAWINGS

In the following detailed description, examples of the present invention are described with reference to the enclosed drawings in which
- FIG. 1: schematically illustrates a device for testing visual processing capabilities of a subject according to an example;
- FIG. 2: schematically illustrates a visual stimulus picture for testing visual processing capabilities of a subject according to an example;
- FIG. 3: is a flow diagram of a method for testing visual processing capabilities of a subject according to an example; and
- FIG. 4: is a flow diagram of a method for testing visual processing capabilities of a subject according to an example.

### DETAILED DESCRIPTION

In the following, detailed examples of the invention are described with respect to a device and method for testing visual processing capabilities of a subject. The approaches disclosed herein generally serve for providing a device and method for testing visual processing capabilities of the subject to indicate and predict an autism status of the subject. More specifically, the approaches disclosed herein may serve for providing an objective, accurate, non-invasive, readily available, and user-friendly test to support decision-making in diagnosing an autism status of a subject.

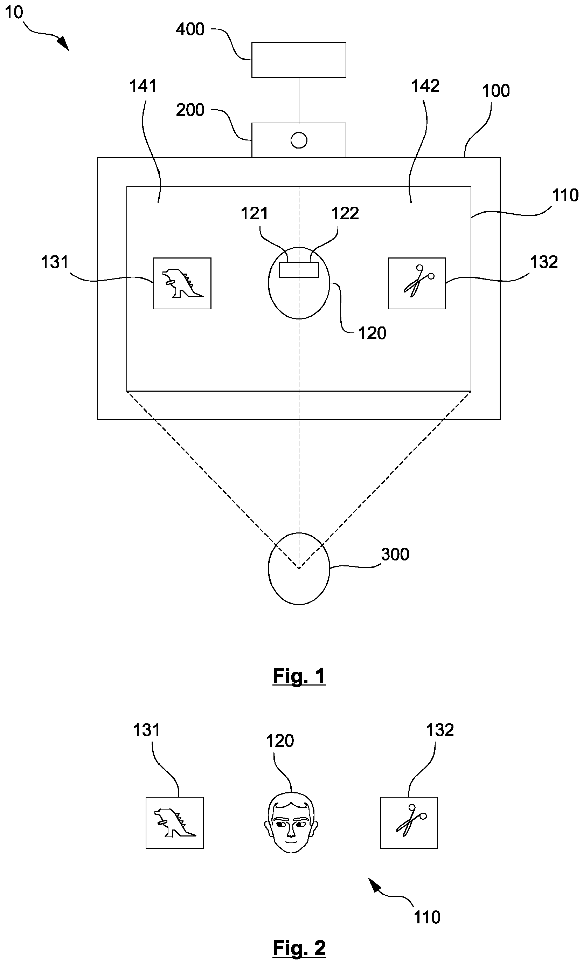

Fig. 1 shows a device for testing visual processing capabilities of a subject according to an example of the invention. The device 10 may comprise a display unit 100 configured to display a visual stimulus picture 110 to the subject 300 and a tracking unit 200 configured to perform eye tracking to record gaze data of the subject 300 during display of the visual stimulus picture 110. The gaze data may include one or more time parameters associated with the gaze of the subject 30 to one or more areas 121,122,131,132,141,142 in the visual stimulus picture 110. The device may further comprise a processing unit 400 configured to evaluate the one or more time parameters in the recorded gaze data. The one or more time parameters included in the gaze data may be capable of indicating an autism status of the subject 300. The evaluated one or more time parameters may be provided to a clinician in order to support his or hers decision-making in diagnosing an autism status of the subject 300.

Additionally, or alternatively, the processing unit 400 may be configured to perform a machine learning algorithm to predict the autism status of the subject 300 based on the evaluation of the one or more time parameters in the recorded gaze data. Machine learning (ML) approaches such as Linear Model, K-nearest-neighbor, Support Victor Machine, Naive Bayes Model, Random Forest Model (RFM), Decision Tree, Artificial Neural Network, and Long Term Short Term Memory (LSTM) may be used. The final outcome was analysed using a confusion matrix. RFM and LSTM models are viable techniques for ASD prediction. The RFM model is preferable since it outperformed the LSTM model.

The display unit 100 may comprise a screen to display the visual stimulus picture 110 to the subject 300. The tracking unit 200 may comprise an optical system, such as one or more cameras, for capturing the gaze of the subject 300 during display of the visual stimulus picture 110 to the subject 300, i.e., recording the gaze data of the subject 300. The processing unit 400 and the tracking unit 200 may be communicatively connected for transferring the gaze data.

The one or more time parameters may include a time between the display of the visual stimulus picture 110 to the subject 300 and a fixation of the gaze of the subject 300 to an area 121,122,131,132,141,142 in the visual stimulus picture 110. Additionally, or alternatively, the one or more time parameters may include a dwell time of the gaze of the subject 300 to the area 121,122,131,132,141,142 in the visual stimulus picture 110.

As shown in Fig. 1 and Fig. 2, the visual stimulus picture 110 displayed to the subject 300 may depict a face 120 and two objects (here a dinosaur toy and scissors) on the left and the right side of the face 120, respectively. However, it is to be understood that the visual stimulus picture 110 may also depict less or more than two objects, or the objects may be in other positions in the visual stimulus picture, e.g., above or below the face 120. The face 120 depicted in the visual stimulus picture 110 may have a gaze direction towards one of the objects as illustrated in Fig. 2. In the example shown in Fig. 2, the face 120 has a gaze direction towards the dinosaur toy on the left side of the face 120. The face 120 may be the face of a human, and more specifically, the face of an avatar of a human child. Alternatively, the face 120 may be the face of a cartoon animal.

The face 120 depicted in the visual stimulus picture 110 may change its gaze direction from one of the one or more objects or events to another. This may be achieved by an animation of the visual stimulus picture 110 or by display of a movie. Additionally, or alternatively, the display unit 100 may be configured to consecutively display a plurality of visual stimulus pictures 110 to the subject 300, wherein each of the plurality of visual stimulus pictures 110 is displayed to the subject for a predetermined duration, e.g., several seconds. For example, each of the plurality of visual stimulus pictures 110 may be displayed to the subject 300 for a duration in the range of 0.5 seconds to 20 seconds, more preferably 1 second to 10 seconds, and more preferably 3 seconds to 5 seconds. The visual stimulus pictures of the plurality of visual stimulus pictures 110 may differ from each other in appearance of the depicted face 120, position, type, or property of the depicted objects, or gaze direction of the face 120. For example, the visual stimulus pictures 110 may differ from each other in the face 120 being male or female, in skin, eye, or hair color, or other facial features. Further, the visual stimulus pictures 110 may differ from each other in the objects being different types of toys (i.e., dinosaur toy, scissor, toy car, doll), or the objects having a different color, size, or position in the visual stimulus picture. Further, the visual stimulus pictures 110 may differ from each other in that the depicted face 120 either gazes directly at the observer, gazes at a specific depicted object, or changes gaze direction to a different object. This may enable to rule out personal preferences of the subject and interferences between the metrics, e.g., joint attention and left visual field bias when an object is positioned to the left or the right side of the face 120 that gazes at the object. Thereby, systematic errors may be excluded, and testing accuracy may be improved.

In an example, at least two time parameters may be evaluated. The at least two time parameters may be the time the subject 300 is viewing an area in the visual stimulus picture located in the left hemifield 141 and the right hemifield 142 of the subject's visual field, respectively. Additionally, or alternatively, the at least two time parameters may be the time the subject 300 is viewing an area 121 in an eye region of the face 120 in the left hemifield 141 of the subject's visual field and an area 122 in an eye region of the face 120 in the right hemifield 142 of the subject's visual field, respectively. Additionally, or alternatively, the at least two time parameters may be the time the subject 300 is viewing an area 131 in a region of the image of the one of the two objects (towards which the gaze of the depicted face 120 is directed) and an area 132 in a region of the image of the other one of the two objects (from which the gaze of the depicted face 120 is averted). A subset or all the above-described parameters may be evaluated jointly for providing an indication for a potential autism status of the subject 300. This may further increase testing accuracy. More specifically, evaluating a subset or all the above-described parameters in a combination may further increase the accuracy for providing an indication for and consequently, for predicting an autism status of the subject 300.

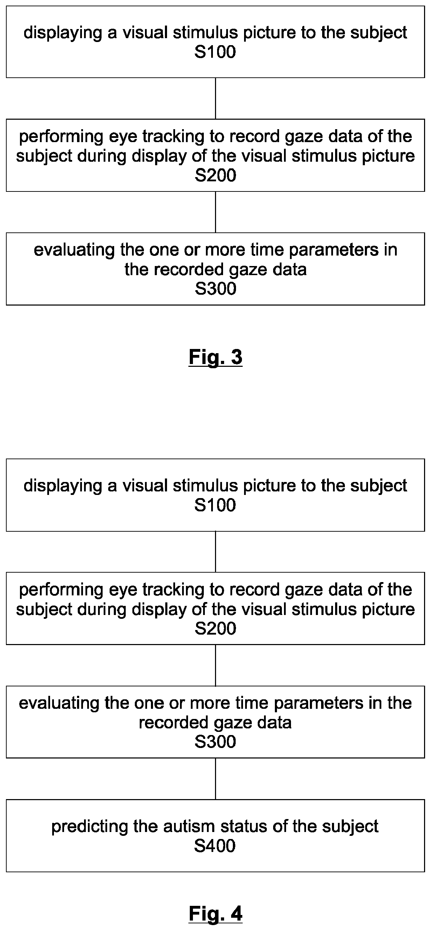

Fig. 3 presents a flow diagram of a method for testing visual processing capabilities of a subject according to an example. The method may comprise a step S100 of displaying the visual stimulus picture 110 (as shown in Fig. 1 and Fig. 2) to the subject 300 and a step S200 of performing eye tracking to the record gaze data of the subject 300 during display of the visual stimulus picture 110. The gaze data may include the one or more time parameters associated with the gaze of the subject 300 to the area 121,122,131,132,141,142 in the visual stimulus picture 110. The method may further comprise a step S300 of evaluating the one or more time parameters in the recorded gaze data. The method presented in Fig. 3 may be used for testing visual processing capabilities of a plurality of subjects divided in two groups of subjects, i.e., with or without autism status. The evaluated one or more time parameters of each individual subject may be used to calculate an average value for the one or time parameters for each group of subj ects. In an example, the average values of the one or time parameters for the group of subjects not having an autism status may be used as standard values for predicting an autism status of a subject in a following test performed according to a method for testing visual processing capabilities as illustrated in Fig. 4.

Fig. 4 presents a flow diagram of a method for testing visual processing capabilities of a subject according to an example. The method may comprise all the steps S100, S200, S300 of the method illustrated in Fig. 3 and may further comprise a step S400 of predicting the autism status of the subject 300 based on the evaluation of the one or more time parameters in the recorded gaze data. More specifically, the autism status may be predicted based on a comparison of the evaluated one or more time parameters in the recorded gaze data with standard values. Additionally, or alternatively, a machine learning algorithm may be performed to predict the autism status of the subject 300.

Many modifications may be made to the above-described examples without departing from the scope of the claims.

### EXAMPLE TESTS

In the following, example tests for visual processing capabilities of subjects employing a device and method according to the present disclosure are described.

Ethical approval for the example tests was obtained from King Faisal Specialist Hospital and Research Centre (RAC - 2201183). The tests took place in the Human Behavior Lab at King Faisal Hospital. A total of 130 subjects were recruited to participate. A number of subjects (N = 26) were excluded from the analysis due to poor calibration or engagements with many stimuli attributes with null values. The total number of subjects with acceptable calibration and engagement rate was N=104 (74 males, 30 females), with 63 subjects found to having an autism status and 41 neurotypical. In the group with autism status, the youngest subject had the age of one year and 10 months and the oldest subject had the age of 22 years; in the neurotypical group, the youngest subject was two years old and the oldest 17 years old. The mean age of the group having an autism status was 8 ± 3.89 years while the mean age of the neurotypical group was 8.21± 4.12 years. The two groups were comparable on both age (t-test; *t* = 0.26₍₇₂₎, *p* = 0.797, *d* = -0.05) and gender (*Xsquared* = 19.36₍₁₀₇₎, *p* = 1).

A Social Communication Questionnaire was collected for most participants of both groups (N=94). Confirmation of an Autism Spectrum Disorder diagnosis was performed in the clinic with a multidisciplinary team that includes five divisions: pediatric neurology, psychology, occupational therapy and sensory integration, speech and language pathology and behavioral analysis and observation.

Visual stimulus pictures were designed to examine visual behavior differences in the tests involving social perception reflected in the three different metrics described herein: left visual field bias, joint attention, and attention to eyes with respect to cartoon faces of humans and animals. Four visual stimulus pictures depicting different avatar child faces having direct gaze towards the observer were used. Each visual stimulus picture was displayed to the subjects for a total of 4 seconds while the gaze data of the subjects were recorded. In addition, corresponding visual stimulus pictures of four animal cartoon faces with direct gaze were used. Moreover, additional visual stimulus pictures similar to the visual stimulus picture 110 shown in Fig. 2 were used. The additional visual stimulus pictures depicted avatar child faces with their gaze directed towards one of the two objects, i. e. target toys, in a numerically balanced and pseudo randomized manner on the left or the right side of the avatar child face. In total, four avatar child identities were presented, and each identity was presented twice with the target toy to the left or the right side. Two types of time parameters were evaluated: time between the display of the visual stimulus picture to the subject and a fixation of the gaze of the subject to the area in the visual stimulus picture, hereinafter abbreviated with TTFF, and dwell time of the gaze of the subject on the target area in the visual stimulus picture.

There were no instructions to the subjects during the task, besides to merely "look at the screen". Other metrics were also examined during the example tests. The tests were ran using iMotions software version 8.3. The display unit 100 was a 24-inch screen with a resolution of 1920×1080 pixels. Areas of interests (AOI) were determined for each displayed visual stimulus picture. An eye tracker by Tobii Pro Fusion was used as tracking unit 200, which captured gaze data at a maximum frequency of 120 Hz.

During the tests, the subjects sat at a constant or substantially constant distance to the screen. The distance for each subject was in the range of 50 cm to 60 cm. The height at which the subjects viewed the screen was standardized by using an adjustable chair and footrest. The average height between the floor and the center of the screen was approximately 114 cm for most of the subjects. The guardians were able to observe the subjects from behind a one-way glass mirror located behind the subjects. Some subjects wanted one guardian to join and sit on their lap. In such cases, the guardian was asked to wear black sunglasses to avoid data contamination. In compliance with the ethical approval obtained for the example test, at least one guardian was present at all times during the test.

The relevant areas of interest (AOIs) were drawn on each of the visual stimulus pictures using the iMotions software. More specifically, for observing and evaluating the time parameters associated with the subject viewing an area in an eye region of a face in visual stimulus picture, i. e. attention to eyes, AOIs were drawn on the eye region of the faces depicted in the prepared visual stimulus pictures with the face having a direct gaze direction towards the observer. For observing and evaluating the time parameters associated with the subject viewing an area in the visual stimulus picture located in the left hemifield of the subject's visual field, i. e. the metric of left visual field bias, AOIs were drawn on both the left and the right sides of the faces having direct gaze in the visual stimulus pictures. For observing and evaluating the time parameters associated with the subject viewing an area in a region of the image of the one of the two objects towards which the gaze of the depicted faces are directed, i. e. the metric of joint attention, AOIs were drawn over both objects, wherein the AOI of the object towards which the gaze of the faces in the visual stimulus pictures is directed to is labelled as "congruent" and the other one of the two objects, which is not gazed at by the depicted faces, is labeled as "incongruent". Then, from the iMotions system, the time parameters were exported for each AOI per subject per visual stimulus pictures. Outliers or subjects with missing data (1-3 per test) were removed in each condition. Finally, the average values were calculated for each group, and dependent or independent t-tests and effect sizes were calculated to measure group differences on the collected metrics. All statistical analysis was done in R (libraries used 'tidyverse', `psych', 'effsize'). For classifying each individual subject (autism status or not), a Random Forest modelling approach based on individual variable features was used. In the model, among other closely related features, the time parameters shown best to differentiate the two groups in t-tests were included, i.e., dwell time and TTFF to the left eye, left side of the face, left side of the screen, for human and animal faces as well as total dwell time and TTFF to the congruent objects of the joint attention task. The entire recorded data set was split in 80% and 20% for training set and validating set, respectively. The total number of features was 154.

**Table 1 Results of the evaluation of the time parameters. TTFF: Time to First Fixation. Statistically significant values are shown in bold.**

| Time Parameter | Autism Status | Controls | Group difference (p value; d value) |
|---|---|---|---|
| | **Left visual field bias** | | |
| Dwell Left - Human | 833.32 ± 416.11 | 959.94 ± 271.31 | **0.04; -0.37** |
| Dwell Left - Animal | 1269.87 ± 830.61 | 1843.9 ± 722.72 | **0.0004;-0.72** |
| Dwell Right - Human | 780.76 ± 368.99 | 770.5 ± 251.53 | 0.87; 0.03 |
| Dwell Right - Animal | 1256.62 ± 743.71 | 1421.68 ± 668.12 | 0.25,0.03 |

| | **Joint attention** | | |
|---|---|---|---|
| Dwell - Congruent | 975-77 ± 447.94 | 1421.17 ± 567.30 | **0.0001; -0.90** |
| TTFF - Congruent | 1097.49 ± 567.49 | 839.33 ± 424.72 | **0.01; 0.5** |
| Dwell - Incongruent | 769.83 ±447.89 | 898.1 ±323.58 | 0.10; -0.31 |
| TTFF - Incongruent | 1133.45 ± 577.64 | 1164.32 ± 485.03 | 0.78; 0.06 |

| | **Attention to the eyes** | | |
|---|---|---|---|
| Dwell on eyes - Human | 1424.93 ± 950.28 | 1554.42 ± 783.78 | 0.46; -0.14 |
| Dwell on eyes - Animal | 1210.68 ± 719.74 | 1456.34 ± 673.09 | 0.09; -0.35 |

The results for the evaluation of the time parameters from the example tests are shown in Table 1. Compared with the control group of subjects not having an autism status, the group of subjects having an autism status had significantly smaller dwell times on the left side of the face for both the human (*t* = 2.05_{(100.9)}, *p* = 0.04, *d* = -0.37) and animal (*t* = 3.66_{(83.65)}, *p* = 0.0004, *d* = -0.72) faces. Dwell time on the right side of the face was not significant across the groups for neither human (*t* = 0.16_{(99.42)}, *p* = 0.87, *d* = 0.03) nor animal (*t* = 1.15_{(84.85)}, *p* = 0.25,*d* = 0.03) faces. Thus, the example tests employing a device and method according to the present disclosure revealed reduced left visual field bias for the group of subjects having an autism status with a moderate to large effect size.

For the congruent objects in the joint attention task (i.e., the objects which are gazed at by the faces in the visual stimulus pictures), the autism group had longer TTFF (*t* = 2.57_{(91.66)}, *p* = 0.01, *d* = 0.5) and a shorter dwell time (*t* = 4.08_{(62.65)}, *p* = 0.0001, *d* = -0.90) than the control group. Effect sizes were large in both cases. By contrast, there were no significant differences in dwell time (*t* = 1.6_{3(91.02)}, *p* = 0.10, *d* = -0.31) or TTFF (*t* = 0.28_{(83.85)}, *p* = 0.78, *d* = 0.06) on incongruent objects. Thus, the example tests employing a device and method according to the present disclosure revealed that the group of subjects with an autism status showed a selective reduction in joint attention.

The two groups showed similar dwell time to the eyes for human faces (*t* = 0.74_{(89.66)}, *p* = 0.46, *d* = -0.14) and animal faces (t = 1.72₍79.48), *p* = 0.09, *d* = -0.35). However, for the visual stimulus pictures with animal cartoon faces, a trend for the autism group to focus less on an area in the eye region could be seen, with a moderate effect size. Hence, displaying a visual stimulus picture depicting the face of a cartoon animal may be particularly suitable for testing for signs of autism.

The employed machine learning algorithm (Random Forest model) provided a prediction of an autism status at the individual level with a sensitivity of 0.82, a specificity of 0.69 and an overall accuracy of 0.71. Hence, the example tests showed that a device and method according to the present disclosure are suitable for predicting an autism status of the subject.

The above-described example tests generally show that a device and method according to the present disclosure is suitable for testing visual processing capabilities of a subject. Moreover, the evaluation of the one or more time parameters according to the above-described example tests was able to provide an objective indication for an autism status of the subjects participating in the tests. Further, an accurate prediction for an autism status of the subjects was achieved.

## Claims

1. Device for testing visual processing capabilities of a subject, comprising
a display unit configured to display a visual stimulus picture to the subject;
a tracking unit configured to perform eye tracking to record gaze data of the subject during display of the visual stimulus picture, the gaze data including one or more time parameters associated with the gaze of the subject to an area in the visual stimulus picture; and
a processing unit configured to evaluate the one or more time parameters in the recorded gaze data, the one or more time parameters capable of indicating an autism status of the subject.

2. The device of claim 1, wherein the one or more time parameters include a time between the display of the visual stimulus picture to the subject and a fixation of the gaze of the subject to the area in the visual stimulus picture or a dwell time of the gaze of the subject on the area in the visual stimulus picture.

3. The device of claim 1 or 2, wherein the one or more time parameters are associated with the subject viewing an area in the visual stimulus picture located in the left hemifield of the subject's visual field.

4. The device of anyone of claims 1 to 3, wherein the visual stimulus picture includes an image of a face, preferably the face of a human or the face of a cartoon animal and wherein the one or more time parameters are associated with the subject viewing an area in an eye region of the face depicted in the visual stimulus picture.

5. The device of anyone of claims 1 to 4, wherein the one or more time parameters are associated with the subject viewing an area in a region of an image of one or more objects or events depicted in the visual stimulus picture.

6. The device of claim 5, wherein a face depicted in the visual stimulus picture has a gaze direction towards one of the one or more objects or events or wherein the face depicted in the visual stimulus picture changes its gaze direction from one of the one or more objects or events to another.

7. The device of anyone of claims 1 to 6, wherein the display unit is configured to consecutively display a plurality of visual stimulus pictures to the subject, wherein each of the plurality of visual stimulus pictures is displayed to the subject for a predetermined duration.

8. The device of anyone of claims 1 to 7, wherein the processing unit is further configured to predict the autism status of the subject and wherein the processing unit is configured to perform a machine learning algorithm to predict the autism status of the subject based on the evaluation of the one or more time parameters in the recorded gaze data.

9. The device of claim 8, wherein the processing unit is configured to predict the autism status of the subject based on comparing the evaluated one or more time parameters in the recorded gaze data to standard values.

10. Method for testing visual processing capabilities of a subject, comprising
displaying a visual stimulus picture to the subject;
performing eye tracking to record gaze data of the subject during display of the visual stimulus picture, the gaze data including one or more time parameters associated with the gaze of the subject to an area in the visual stimulus picture;
evaluating the one or more time parameters in the recorded gaze data, the one or more time parameters capable of indicating an autism status of the subject.

11. The method of claim 10, wherein the one or more time parameters include a time between the display of the visual stimulus picture to the subject and a fixation of the gaze of the subject to the area in the visual stimulus picture or a dwell time of the gaze of the subject on the area in the visual stimulus picture.

12. The method of claim 10 or 11, wherein the one or more time parameters are associated with one or both of the subject viewing an area in the visual stimulus picture located in the left hemifield of the subject's visual field or the subject viewing an area located in a region of an image of one or more objects or events depicted in the visual stimulus picture, wherein a face depicted in the visual stimulus picture has a gaze direction towards one of the one or more objects or events.

13. The method of anyone of claims 10 to 12, comprising consecutively displaying a plurality of visual stimulus pictures to the subject, wherein each of the plurality of visual stimulus pictures is displayed to the subject for a predetermined duration.

14. The method of claim anyone of claims 10 to 13, further comprising predicting the autism status of the subject, wherein the autism status of the subject is predicted based on automatically comparing the evaluated one or more time parameters in the recorded gaze data to standard values.

15. The method of claim 14, wherein a machine learning algorithm is performed to predict the autism status of the subject based on the evaluation of the one or more time parameters in the recorded gaze data.
